# EUROPEAN PATENT SPECIFICATION

(11) **EP 4 142 588 B1**
(45) Date of publication and mention of the grant of the patent: **31.07.2024**
(21) Application number: 21723208.1
(22) Date of filing: 30.04.2021
(51) Int. Cl.: A61B 5/15, A61B 5/153, A61M 25/00, A61M 25/01, A61M 25/06

(54) **CATHETER ASSEMBLY**
KATHETERANORDNUNG
ENSEMBLE CATHÉTER

(30) Priority: 01.05.2020 US 202063019174 P
(43) Date of publication of application: 08.03.2023
(73) Proprietor: B. Braun Melsungen AG, 34212 Melsungen (DE)
(72) Inventor: TEOH, Hui Kuun, Penang, 10810 (MY); PHANG, Chee Mun, Penang, 10810 (MY); ANG, Woon Chau, Penang, 10810 (MY); ONG, Eng Keat, Penang, 10810 (MY); ABU BAKAR, Bakhtiar, Penang, 10810 (MY); YAAKOB, Yusri, Penang, 10810 (MY)
(74) Representative: Kinkeldey, Daniela
(86) International application number: PCT/EP2021/061443
(87) International publication number: WO 2021/219867

(56) References cited:
- WO-A1-00/13728
- WO-A1-2015/153976
- WO-A1-2019/201859
- WO-A2-2007/022373
- CH-A2- 701 349
- US-A1- 2018 140 240
- US-A1- 2018 214 682

## Description

### Field of Art

The present invention relates to a catheter assembly according to the preamble of claim 1. Such a catheter assembly is known from WO 2019/201859 A1.

### Background

Cannulation is often a daunting task for both the medical professional and the subject. It requires a certain amount of skill and experience on the part of the medical professional to administer the process with as little pain or discomfort to the subject as possible.

Some cannulation systems include a way for the medical professional to visualize primary flashback during the cannulation process. For example, some systems include a notched needle which is surrounded by a catheter during insertion. The notch allows blood to flow into the annular space between the needle and the catheter tube so that the medical professional can observe when primary flashback occurs to understand when the bevel of the needle has entered a vein.

To incorporate notches into metal needles requires an additional processing step over forming a closed-circumference, cylindrical-bodied needle. Additional processing steps complicate the process and require more time and manufacturing expense. Moreover, blood exiting through the notch may lead to blood exposure upon needle withdrawal.

Metal is also the preferred material for conventional cannulation systems due to the low penetration force when compared with needles formed from other materials. The low penetration force enables the needle to more easily enter the vasculature of a patient, thus puncturing into the vasculature with minimal patient discomfort. Document WO 2007/022373 A2 discloses medical devices with needle safety shielding. Document WO 00/13728 A1 discloses an intravenous infusion needle with soft body. Document WO 2019/201859 A1 discloses catheter assemblies and related methods.

It is generally desirable to overcome or ameliorate one or more of the above described difficulties, or to at least provide a useful alternative.

### Summary

In accordance with the present invention, there is provided catheter assembly comprising: a needle, a catheter hub; and a catheter connected to the catheter hub and surrounding the needle, wherein the needle comprises: a needle tip formed from a first rigid material, the needle tip comprising a bevel for puncturing a subject; and a needle shaft comprising a second material, wherein the needle tip is separately formed and subsequently attached to the needle shaft to form the needle; wherein the second material is a light permeable material to enable visible detection of primary flashback in the needle shaft. The first material can have higher rigidity than the second material.

The first material can be a metal.

The inner material having the needle tip can be a substrate upon which the second material is attached, moulded thereto or deposited thereon.

In an example, the substrate can be made from a non-metallic material and a harder metallic material can be deposited onto the substrate.

In some examples, the substrate can have both a shaft section and a needle tip section having a needle bevel. The shaft section can attach to the needle tip section via a coupling.

The second material can be a plastic material.

The second material can be a light permeable material to enable visible detection of primary flashback in the needle shaft. The primary flash back can be viewed looking through the needle formed using the second material. When used with a catheter, blood flow can flow in the annular space between the catheter tube and the needle and can block the needle from observation. The flow in the annular space can be secondary flashback.

The second material can be transparent or translucent.

The needle tip can attach to the needle shaft through a coupling. The coupling can have a first coupling part formed with the needle tip and a second coupling part formed with the shaft section. The first coupling part can couple with the second coupling part to form a completed or assembled coupling to attach the needle tip to the needle shaft.

The coupling can comprise: one or more indentations on one of the needle shaft and the needle tip; and, for each indentation, a protrusion on the other one of the needle shaft and the needle tip, and arranged to be received in the indentation, thereby to couple the needle tip to the needle shaft.

The coupling can comprise: one or more apertures on one of the needle shaft and the needle tip; and for each aperture, a protrusion on the other one of the needle shaft and the needle tip, and arranged to be received in the aperture, thereby to couple the needle tip to the needle shaft.

The needle tip can comprise: the first material having the bevel; and either: the second material is formed onto the first material, thereby to form the needle shaft; or the needle shaft is formed from the second material and thereafter connected to the first material.

The needle shaft can comprise an integral needle tip at a distal end of the needle shaft, and the needle tip can attach to the needle shaft by depositing the first material over an integral needle tip form.

The needle tip can comprise: a proximal end comprising one of a depression and protrusion; and wherein forming the second material onto the needle tip can comprise depositing the second material over a length of the needle tip from a position distal of the depression or protrusion to, and past, the proximal end of the needle tip.

The needle can comprise an intermediate material formed on, or attached to, the integral needle tip form for heat dissipation during depositing the first material. Said differently, the needle can be made from a first material. An intermediate layer can form over the first material. A second material can form over the second material. The three materials can form a needle with a needle tip of the present invention.

The second material can be molded onto the first material.

The first material can bound to the second material during cooling or curing of the second material.

The needle tip can comprise an aperture to form a window for viewing primary flashback. For example, where the needle is formed by coating a second material over a first material, a portion or section of the inner first material can be masked so that when the first material is located over the second material, the masked part of the first material can function as an observation window to observe primary flashback. The window can have a solid non-removable surface that is transparent or semi-opaque.

A needle hub can attach to the needle. A tether can connect between the needle tip and needle hub to couple the needle tip to the needle hub.

In some examples, a needle with a needle tip can be secured to the needle hub via a needle weld. The needle can have at least two different materials. The second material can be deposited onto the first material or moulded to the first material, such as inside the first material or outside the first material.

The needle shaft can comprise a marking, the marking can be located on the needle shaft to be visible during primary flashback.

The catheter assembly can comprise a valve and a valve opener located inside the interior of the catheter hub.

The valve can comprise a plurality of slits defining a plurality of flaps. In an example, the valve can comprise three slits and three flaps.

The valve opener can comprise a nose section and two plunger elements extending proximally of the nose section. In an example, two stabilizer elements can each connect to the two plunger elements. The two stabilizer elements and the two plunger elements, at the connection region, can define a stabilizing ring.

Two through openings can be provided between the nose section and the stabilizing ring. In an example, the needle guard can comprise two elbows. Each elbow can locate in a respective through opening of the valve opener.

The catheter assembly can have a needle guard located in the interior of the catheter hub.

The needle guard can be located between two plunger elements of the valve opener.

Methods of making and of using the needles, needle devices, and catheter devices and components thereof are disclosed herein, however, not claimed.

### Brief Description of the Drawings

Preferred embodiments of the invention are hereafter described, by way of nonlimiting example only, with reference to the accompanying drawings, in which:
Figure 1 is a needle;
Figure 2 is a section view through the needle shown in Figure 1;
Figure 3 is a cannula including the needle shown in Figure 1;
Figure 4A is an exploded view of a needle;
Figure 4B is the assembled needle shown in Figure 4A;
Figure 5 is a section view of a needle assembly including the needle shown in Figure 1;
Figure 6A is an exploded view of a needle;
Figure 6B is the assembled needle shown in Figure 6A;
Figure 7A is an exploded view of a needle;
Figure 7B is a section view of the assembled needle shown in Figure 7A;
Figure 8A is an exploded view of a needle;
Figure 8B is a section view of the assembled needle shown in Figure 8A;
Figure 8C is the assembled needle shown in Figure 8A;
Figure 9A is an exploded view of a needle;
Figure 9B is a section view of the assembled needle shown in Figure 9A;
Figure 9C is the assembled needle shown in Figure 9A;
Figure 10 is another embodiment of a needle;
Figure 11 is a catheter assembly, showing a catheter tube located over a needle;
Figure 12 illustrates an alternative embodiment of a needle formed from multiple materials;
Figures 13A to 13D illustrate various methods of forming a needle in accordance with present teachings; and
Figure 14 illustrates a catheter assembly, a needle hub, and a needle according to present teachings.

### Detailed Description

The detailed description set forth below in connection with the appended drawings is intended as a description of the presently preferred embodiments of needles and catheter assemblies incorporating the needles and components thereof provided in accordance with aspects of the present devices, systems, and methods and is not intended to represent the only forms in which the present devices, systems, and methods may be constructed or utilized. The description sets forth the features and the steps for constructing and using the embodiments of the present devices, systems, and methods in connection with the illustrated embodiments. It is to be understood, however, that the same or equivalent functions and structures may be accomplished by different embodiments that are also intended to be encompassed within the scope of the present disclosure. As denoted elsewhere herein, like element numbers are intended to indicate like or similar elements or features. The invention is determined by claim 1.

Described herein are mixed material needles and catheter assemblies that use mixed material needles. The mixed material needles described herein enable provision of rigid, low penetration force material at the bevel of the needle, and a different material along the shaft or shank of the needle. Thus, the benefit of low penetration force is retained, while enabling the shaft to be formed from a more cost-effective material, a material that is more readily formed into a cylindrical needle shaft, and/or a material having different, but advantageous, properties, when compared with metal needle shafts.

Advantageously, the bevel of the needle at the needle tip being of a rigid material, such as metal, results in lower deformation of the needle assembly during penetration. Further, it is easier to fabricate a small diameter metal bevel as compared to plastic, for example. It is also easier to obtain a sharp edge on a metal bevel, for example by grinding or machining, as compared to plastic for example.

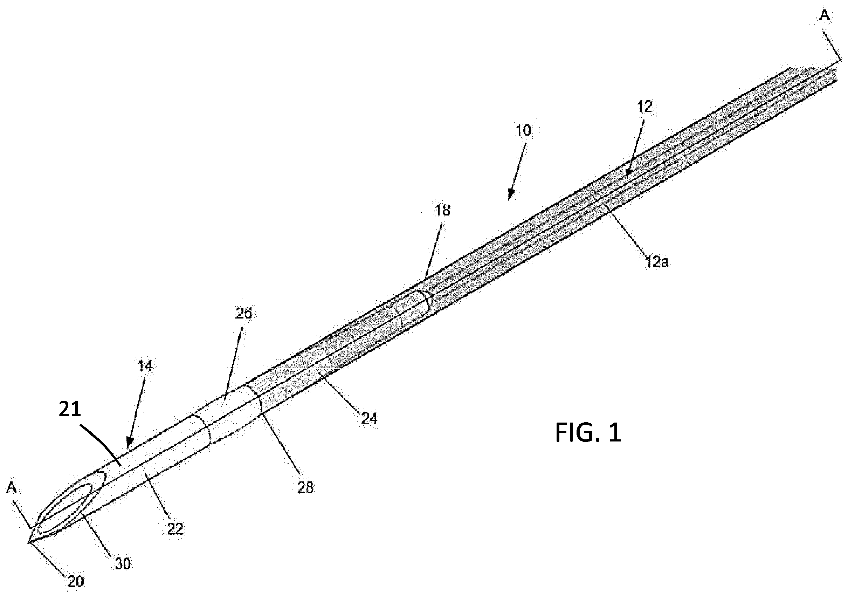

With reference now to Figure 1, an embodiment of a needle 10 in accordance with aspects of the invention is shown. The needle 10is suitable for intravenous catheterisation, a hypodermic needle, biopsy needle, central venous catheter (CVC) needle, blood collection needle, dialysis fistula needle, and spinal needle, among others. It will be appreciated that the present teachings can be applied broadly to needles for medical procedures and are not limited to applications described with reference to the examples herein, nor to specific gauges or lengths. The needle 10 broadly comprises a sharpened needle tip 14 and a needle shaft 12. In exemplary embodiments, the needle tip 14 is separately formed and attachable to the needle shaft 12. In use, the needle tip 14 is attached to the needle shaft 12 along an interface or attachment point to form the needle 10.

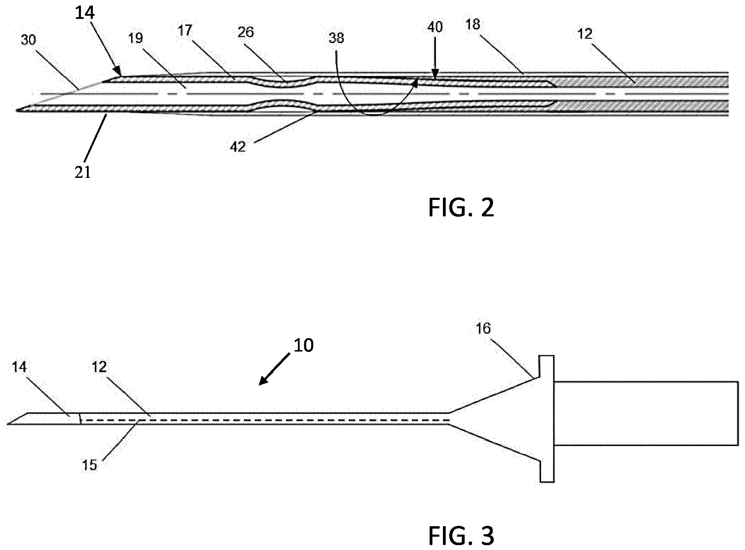

The needle 10 may be formed by one or more materials, such as plastic and metal. Moreover, the needle shaft 12 may be defined by the length of the needle between the bevel 30 and the point at which the needle is grasped during use, or the needle hub if the needle hub is grasped. Thus, the shaft length can be a variable depending on where the needle is grasped. The needle shaft 12 may similarly be defined by the length between the bevel 30 and the needle hub 16 (Figure 3). In a specific example, the needle shaft 12 is defined by the length between the bevel 30 and the proximal end of the needle.

Where the needle 10 is moveable with the needle hub 16, the needle 10 may have an extended condition in which the needle 10 is in position to penetrate a patient, and a retracted condition in which the needle 10 is retracted from the patient. For example, after penetration of a patient, a catheter can advance over the needle 10 and the needle retraced inside the catheter and eventually completely separated from the catheter. The needle shaft 12 may comprise a distal length 12a and a proximal length 12b. The distal length 12a can be the length between the bevel 30 and the hub 16. The distal length 12a may be of sufficient rigidity to support the needle tip 14 during insertion thereof into a patient, e.g., without substantial flexing or bending in the needle shaft. Excessive level of flexing or bending can cause noticeable discomfort. The needle shaft 12 may also comprise a proximal length 12b extending proximally from the distal length 12a. The distal length and the proximal length together defining the needle shaft. Thus, reference to the needle shaft may refer only to the distal length 12a, or to both the proximal 12b and distal 12a lengths.

The needle tip 14 and needle shaft 12 may be supplied separately and assembled in a hospital or clinic, or immediately before use. Alternatively, the needle 10 may be supplied as an assembled product in which the needle tip 14 is attached to the needle shaft 12 at an attachment point as shown. The needle 10, with the needle shaft 12 and the needle tip 14, can be hollow or solid, such as a stylet.

In an example, the needle tip 14 is a hollow, cylindrical body with a needle bevel at a distal end. The needle tip 14 has a distal end 20 with a pointed tip, a bevel 30, and a cylindrical portion 21. The needle tip 14 be thought of as comprising a distal portion 22 and a proximal portion 24. The distal portion 22 includes the distal end 20 and the cylindrical portion 21.

In some embodiments, the proximal portion 24 comprises a change in profile 26, presently in the form of an indent, depression, or crimp as particularly shown in Figures 1 and 2. Advantageously, the change in profile 26 is shaped to bear against a needle guard, such as against a perimeter defining an opening on the needle guard, to secure the needle guard at the needle tip and to retract the needle guard with the needle tip following successful venipuncture so as to cover the needle tip to prevent inadvertent needle sticks. Exemplary needle guards and engagement between the change in profile 26 and the needle guard are disclosed in U.S. Pat. No. 6,616,630 and in U.S. Pub. No. 2018/0214682. The crimp 26 may also be referred to as an indent or squeeze

The needle tip 14 has an elongate body. In an example, the elongate needle tip 14 tapers proximally or in the proximal direction. For example, the elongate body of the needle tip 14 can have an outer diameter that increases in size in the proximal direction. In some embodiments, the taper may guide insertion of the needle tip 14 into the catheter 18 prior to use. The proximal portion 24 of the needle tip 14 can at least partially be received within the catheter 18 before the catheter 18 is advanced over the needle 10 into the subject. In some examples, the distal end of the catheter 18 has a taper to guide or facilitate insertion of the over-the-needle into the patient. The opening at the distal end catheter can form a tight fit around the needle tip to facilitate insertion.

For embodiments discussed below where the needle tip 14 is assembled to the needle shaft, the needle shaft 12 can at least partially project into the needle tip 14, or the needle tip can at least partially surround the needle shaft. In other examples, the needle tip 14 can have a proximal taper utilized for insertion into the needle shaft 12. In some embodiments, the taper for assembly is only provided within the proximal portion 24 of the needle tip 14.

The sharpened needle tip 14 includes a bevel 30 at the distal end 20, as previously discussed. The bevel 30 may embody a standard needle bevel as known in the art. Alternatively, the distal end 20 may have an alternative needle tip, such as a coring needle tip, different cut bevels, or a blunt needle tip as suitable for some applications. Where the needle tip 14 is made from a metal material and has both a bevel and a cylindrical portion, the needle tip 14 is shorter than conventional metal needles, such as being shorter than 38mm. The needle tip 14 can couple to a plastic needle hub 16 via a needle shaft 12. Preferably, the length of the needle tip 14, including the shape of the bevel, provides a low penetration force, by having sufficient length to maintain rigidity during penetration, which can reduce patient pain or discomfort during penetration.

The sharpened needle tip 14 is formed from a first rigid material. The term "rigid" in this context means that the material is more rigid than the needle shaft 12, or more rigid than plastics materials. The first rigid material can retain the sharp edge and shape of the bevel 30 during penetration of the subject. The first material therefore experiences very little, if any, deformation at the puncture site during penetration.

The needle shaft 12 is formed from a second material. The second material may be the same as the first material, i.e., the material from which the needle tip 14 is formed. However, in embodiments described herein the second material is different from the first material. For example, the second material may be a plastic material or another material of lower rigidity than the material used to form the needle tip 14. The first and second materials may also be selected to facilitate separation of the needle tip 14 and the needle shaft 12. For example, the shaft may be formed from a plastics material that can be heated, such as in an autoclave, to become supple or to become a liquid, to facilitate separation of the needle tip from the shaft. One or both of the first material and the second material may instead be readily soluble.

The needle shaft 12 may be formed from a material of sufficient rigidity to support the needle tip 14 during penetration of the needle tip into a patient, without substantial deformation of the needle shaft 12. In some embodiments, the first material is a metal material and the second material is a plastic material. In some embodiments, the needle shaft 12 includes a spine 15 (Figure 3), or a lengthwise rib, to increase its rigidity. When incorporated, the spine 15 could be added during plastic extrusion, for example, or by over-moulding when over-moulding the needle shaft to the sharpened needle tip is practiced.

In an example, the process of over-moulding comprises forming a part, such as the needle shaft, onto another part, such as the needle tip, by using said another part as part of the mould. For example, the needle tip may be formed from a first material, such as from a metal material, and shaped to form a mould for a portion of the needle shaft. Thus, a mould may comprise the needle tip as an outer part of the mould, and a cylindrical member or mandrel as the inner part of the mould such that a mould cavity is created therebetween. A second material may then be flowed or injected into the cavity and solidified to form part or all of the needle shaft. After curing or setting of the second material, the cylindrical member or mandrel is then removed, resulting in a hollow needle tip with over-moulded needle shaft.

In some cases, the cylindrical member or member is not required, such as where a solid needle is desired rather than a hollow needle. Where a solid needle is formed, the needle tip may form a closed end or a solid body (i.e., not hollow) even before the shaft is moulded onto the needle tip. Alternatively, the needle tip may be hollow and the shaft fills the internal volume of the needle tip during moulding. The needle tip can then be machined to form the sharp needle tip, which also results in shaping the distal end of the shaft as part of the sharpened solid needle tip. In other embodiments, the needle tip and a separately formed needle shaft are connected by adhesion/gluing, heat sealing, ultrasonic welding, interference fit or any other appropriate attachment means. In yet another embodiment, the needle is formed entirely of the second material, such as from a plastic material, and the needle tip is coated into or onto the shaft, such as by plating or electroplating metal onto the tip, to form a tip having a more rigid property than the non-coated part of the needle.

The term "under-moulded" may be used to refer to the same over-moulding process described hereinabove, but recognize that the needle shaft is formed under rather than over or around proximal end of the needle tip. In an alternative embodiment, the needle tip may be rolled from a flat shape into a cylindrical shape onto the needle shaft.

In some embodiments, the needle 10, in particular the needle tip 14, is coated. For example, the needle 10 is at least partially coated with an antimicrobial agent to reduce occurrences of infections at the penetration site. Antimicrobial agents useable herein include silver, gold, platinum, copper, and zinc. Antimicrobial metal compounds used herein include oxides and salts of preferably silver and also gold, for example silver acetate, silver benzoate, silver carbonate, silver citrate, silver chloride, silver iodide, silver nitrate, silver oxide, silver sulfa diazine, silver sulfate, gold chloride and gold oxide. Platinum compounds such as chloroplatinic acid or its salts (e.g., sodium and calcium chloroplatinate) may also be used. Also, compounds of copper and zinc may be used, for example: oxides and salts of copper and zinc such as those indicated above for silver. Single physiological, antimicrobial metal compounds or combinations of physiological, antimicrobial metal compounds may be used. Still alternatively, a thin antimicrobial agent may be deposited over a wall surface of the needle. Another example of a coated needle 10 is echogenic coating for improving ultrasound visibility. The needle tip 14 may also, or in addition thereto, comprise markings or etchings to improve ultrasound visibility. In other embodiments, a lubricious coating is applied to the needle tip for reducing pain perceived by a patient during penetration.

Advantageously, the second material of the present embodiment is a light permeable material to enable visible detection of primary flashback in the needle shaft. In some embodiments, the second material is translucent or semi-opaque to enable visible detection of when blood is within the needle shaft 12. The second material may instead be transparent.

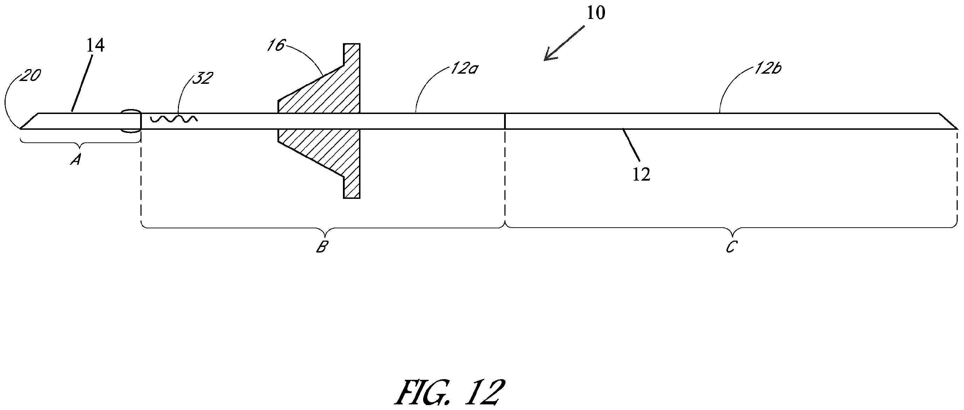

The needle shaft 12 may comprise more than two materials or may change in composition along a length of the needle shaft 12. With reference to Figure 12, a needle 10 with two or more materials or compositions is shown. The needle 10 may comprise three or more distinct Regions A, B, C, each formed from a different material. In certain embodiments, the Regions A, B and C have respectively different diameters, such as different external diameters, different internal diameters, and/or different shaft wall thicknesses. This may result in a different hardness across the different regions. For example, Region C may have a smaller diameter than Regions A and B. In cases where, for example, Region C extends through a septum, the reduced diameter relative to Regions A and B reduces the amount of space occupied by the needle shaft where it projects through the septum. By reducing the shaft diameter, the cross-sectional space inside the catheter hub is freed up by the corresponding amount. This increases the ease with which other components, such as a needle guard, can be mounted into the interior of the catheter hub during assembly.

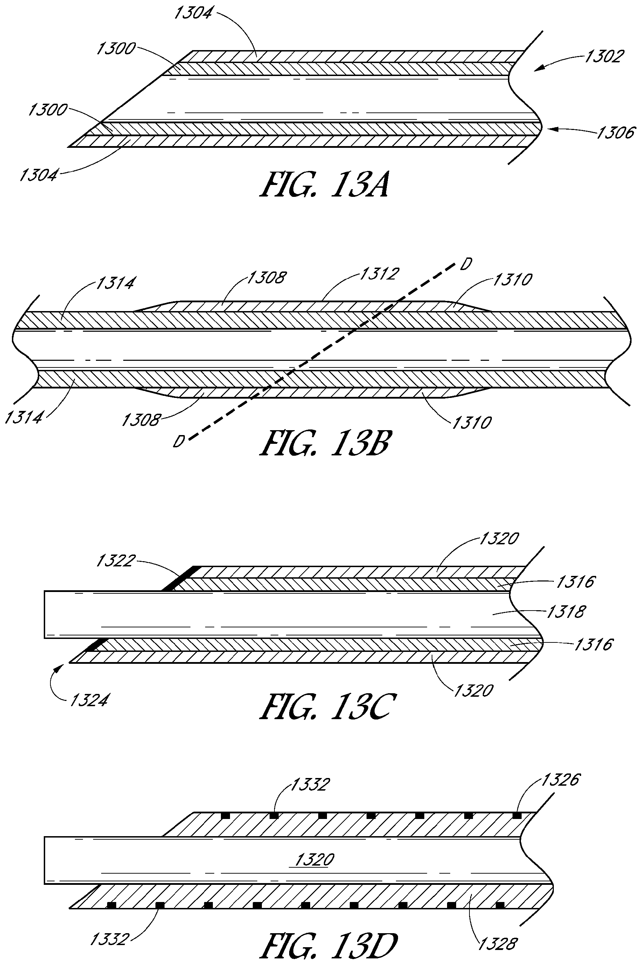

Region A may comprise a needle tip 14 formed from metal. In some examples, the needle tip 14 may comprise a substrate material 1306, as shown in Figure 13A. The substrate material 1306 may have the shape of a needle tip form. That is, the substrate material 1306 can have the shape of a tubular section and a bevel section of a needle tip, called a needle tip form 1306, or a substrate having both a tubular section and a bevel section. The needle tip form 1306 of the substrate material provides the shape over which a first material can be deposited to have a final shape of a needle tip 1304, which has a tubular section and a bevel section of the substrate and of the first material deposited thereon to form a final needle tip 1304. For example, the first material can be deposited onto the substrate material 1306, such as through plating, electrolysis, electrolytic plating, or vapour deposition. Thus, the substrate material 1306 can have an exterior material onto which the substrate material can be deposited. The substrate material 1306 may be a plastic material, such as medical grade silicon.

With reference again to FIG. 12, in some examples, the needle shaft 12 may comprise an integral needle tip form at a distal end of the elongated tubular section of the needle shaft. For example, the substrate material onto which a first material can be deposited may comprise the distal end of the needle shaft 12 and the needle tip 14, or where the needle shaft and the needle tip form of the substrate may be attached as described elsewhere herein, and the first material may be deposited over the integral needle tip and shaft or the substrate may be over-moulded onto a pre-formed needle tip.

Region B may comprise a rigid plastic material, such as a transparent material for primary flashback visibility. Region B may be the distal end 12a of the needle shaft 12. Region C may be formed using a relatively more flexible plastic material and may be referred to as the proximal end 12b' of the needle shaft 12. Notably, a different material may comprise a different composition of the same material, such as low-density polyethylene and high-density polyethylene, a different colour of material, different light permeability and so forth.

Region B may extend in the proximal direction from Region A of the needle 10 to the needle hub 16. Region C may then form a proximal part of the needle 10 that is located proximally of the needle hub 16. In other examples, instead of having discrete changes in material over distinct or discrete regions of the needle 10, the needle 10 may instead comprise one or more regions of continuous change in material, such as a continuous spectrum ranging from most rigid to least rigid. For example, the needle 10 may be formed from high-density polyethylene at its distal end 20 to low density polyethylene at its proximal end located in, or proximal of, the needle hub 16.

In another embodiment, the proximal end of the needle shaft 12, i.e., a proximal end of Region C as particularly shown in Figure 12, is sharpened for easy penetration into a blood collection tube, for example. In other words, the needle 10 of Figure 12 may be practiced as a needle with a distal needle tip and a proximal needle tip, or a needle with two sharpened needle tips. Thus, Region C, in the present embodiment with two needle tips, may be formed from a material of sufficient rigidity so that a sharped proximal tip has the capability of puncturing a septum of a blood collection tube, otherwise known as a vacutainer. In addition, where Region B defines a region of the needle shaft extending from the needle tip 14, and Region C defines a region of the needle shaft extending distally from, and including, the proximal end of the needle shaft, there may be additional regions between Regions B and C, such as Regions D, E, and F, or additional regions.

The needle shaft 12 as particularly shown in Figure 12 comprises markings, herein after referred to as indicia 32. The marking 32 of the present embodiment produces a contrasting colour or appearance when exposed to blood to enable easy visual identification of the indicia. It will be appreciated that one or more indicia may be provided with each needle, each having the same function of the indicia 32 described herein. The one or more indicia enable visible distinction between primary flashback, indicating entry of the bevel 30 into a vein, and secondary flashback, indicating entry of the catheter 18 into that same vein. The indicia 32 are visible during primary flashback when blood flows through the needle lumen. During secondary flashback, blood in the annular space between the outer surface of the needle shaft 12 and internal surface of the catheter 18 (see Figure 1) obscures the indicia 32. Thus, a visible distinction can be made whereby if blood is viewable within the catheter 18 but the indicia 32 are still visible, then that indicates primary flashback. But when the indicia 32 are no longer visible, then that indicates secondary flashback. The indicia 32 may be marked by means of laser marking, pad printing or silk screen printing.

In an example, a needle 10 can be fabricated by attaching a needle tip 14 to a needle shaft 12 using a coupling, as further discussed below. With reference to Figure 2, an internal surface 38 of the needle shaft 12 conforms to an external surface 40 of the needle tip 14 when the needle tip 14 and needle shaft 12 are coupled together. At the interface 42 between the needle shaft 12 and needle tip 14, an outer surface of the interface 42 is of constant diameter or is otherwise smooth. In some embodiments, the outer diameter of the cylindrical portion 21 of the needle tip 14 may be large than outer diameter of the needle shaft 12. In other examples, the outer diameter of the cylindrical portion 21 of the needle tip 14 may be smaller than the outer diameter of the needle shaft 12. The transition from the needle tip 14 to the needle shaft 12, i.e., as a puncture moves from the diameter of the needle tip 14 to the diameter of the needle shaft 12, is ideally sufficiently smooth to ensure the needle shaft 12 does not catch on the tissues of the subject during insertion of the needle 10 into the subject, and ensures that the needle tip 14 does not catch on the tissues of the subject during retraction following successful injection or venipuncture.

In some embodiments, such as that shown in Figure 2, the needle tip 14 comprises a metal tube 17, which has a hollow cavity 19. The catheter 18 may be located externally of the needle, and the metal tube. In an example, the catheter 18 may be held against the outer surface of the metal tube 17 and/or the needle shaft 14 by frictional engagement per known catheter assemblies to prevent catching on human tissues.

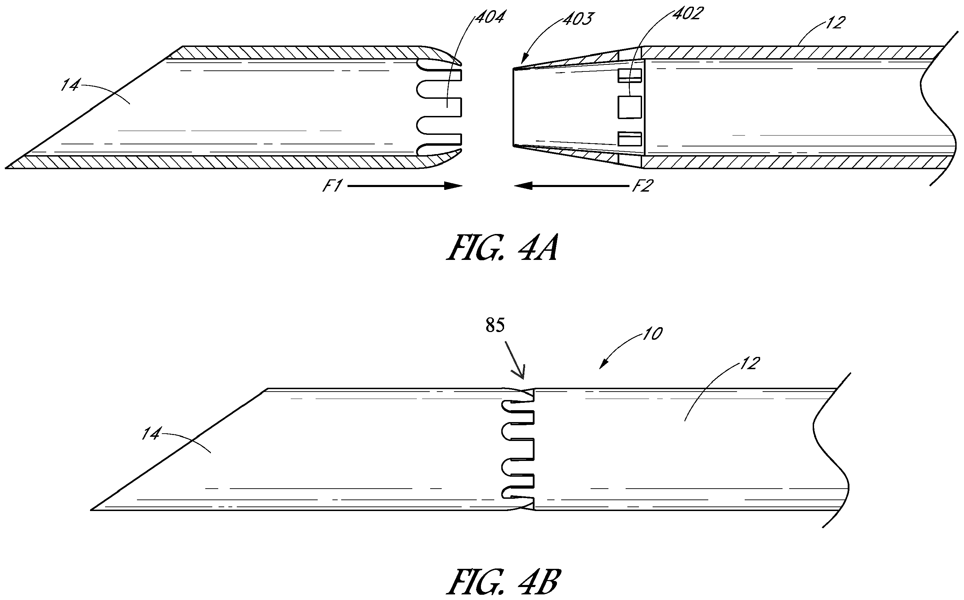

Various forms of coupling may be used for coupling a needle tip 14 to a needle shaft 12 in accordance with aspects of the invention. The particular coupling may be selected to maintain the requisite sterility for a particular use or application of the needle. The coupling may comprise one or more indentations on one of the needle shaft 12 and needle tip 14 and, for each indentation, a complementary protrusion on the other of the needle shaft 12 and needle tip 14. Each protrusion is arranged to be received in a corresponding indentation, thereby to couple the needle tip 14 to the needle shaft 12. For example, for the embodiment shown in Figure 4a, the needle shaft 12 includes one or more recesses or channels 402. The channels 402 are preferably located adjacent to the distal end 403 of the needle shaft 12.

In the embodiment of Figure 4a, the needle tip 14 includes a plurality of projections, presently in the form of metal bevel claws 404. The claws 404 are preferably located at the proximal end of the needle tip 14 and are formed to fit within the complementary channels 402 of needle shaft 12. To couple the needle shaft 12 with needle tip 14 to form needle assembly 10 as particularly shown in Figure 4b, the two components are forced toward each other, in direction F1 and F2 respectively as shown in Figure 4a, until the claws 404 are engaged within the channels 402 as shown in Figure 4b thereby forming a coupling 85 to attach the needle tip 14 with the needle shaft 12. The claws 404 may be biased radially inwardly. During insertion of the distal end 403 of the shaft 12 into the needle tip 14, the claws 404 are urged radially outwardly against the bias of the claws 404, until the claws 404 align with the channels 402. The claws 404 then bias or spring back into the corresponding channels 402 to couple the needle tip 14 to the shaft 12. As shown, the distal end of the shaft 12 can be tapered to facilitate insertion into the needle tip.

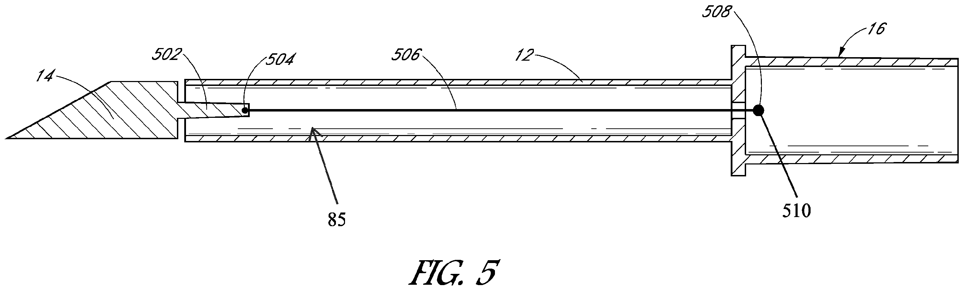

In another embodiment as particularly shown in Figure 5, the needle tip 14 includes a proximally extending projection 502. The proximally extending projection 502 is located at the proximal end of the needle tip 14. In this embodiment, a wire, a thread or a tether 506 is used to secure the needle tip 14 to the shaft 12 or hub 16. Preferably, the one end of the tether 506, such as a first end, is welded, tied or otherwise attached to the projection 502 adjacent the proximal end of needle tip 14, or inserted through an eye 504 in the tip 14 and heated so as to form a bundle. That end of the tether 506, namely the distal end, may alternatively be tethered to the needle tip 14 by a crimp, such as the crimp used to prevent the needle 10 from drawing entirely through a needle guard, and by heating the tether as discussed below to form a bundle that is larger than will fit through the crimp.

Preferably, the opposite end of the tether 506, such as the second end, is coupled to the plastic needle hub 16 at point 508, for example, as particularly shown in Figure 5. The point 508 comprises a slit or a hole having a width or diameter that is equal to or greater than the diameter of the tether 506, but smaller than that of an enlarged section 510 on the second end of the tether 506. In some embodiments, to secure the tether 506 to the plastic hub 16, the proximal end or second end of the tether 506 is bundled or crimped to form the enlarged section 510, which sufficiently large to move back out distally through point 508. For example, the tether 506 may be heated up to form a bundle - some plastics are known to automatically bunch up when heated - thereby coupling the tether 506 to point 508 so as to couple the needle tip 14 to the plastic hub 16. When the tether 506 is entangled at the first end to the needle tip 14 and the second end to the hub 16, the two are coupled via the coupling 85 provided by the tether, which reduces the likelihood of disengagement of the needle tip 14 from the needle assembly 16 when the needle is removed from the patient is reduced.

With reference to Figure 3, a needle assembly is shown. The needle assembly has a needle tip 14 and a needle shaft 12 coupled with a needle hub 16. In an example, the needle shaft 12 is moulded or formed with the needle hub 16 to form a single, unitary component between the shaft and the hub. Thus, when moulded as described, the needle hub 16 may comprise part of the needle, may be attached to the needle, or is moulded with the needle. The needle hub 16 may otherwise be a standard needle hub and comprises an elongated body for gripping. The body of the needle hub 16 may also include a hollow interior and the open proximal end may be provided with a hydrophobic filter.

In other examples, the plastic needle hub 16 may be coupled to the needle shaft 12 to form the needle 10 by mechanical or securement means. In some examples, the means for securing, such as a coupling, includes an interference fit, friction fit, ultrasonic welding, adhesive or bonding, and other attachment methods.

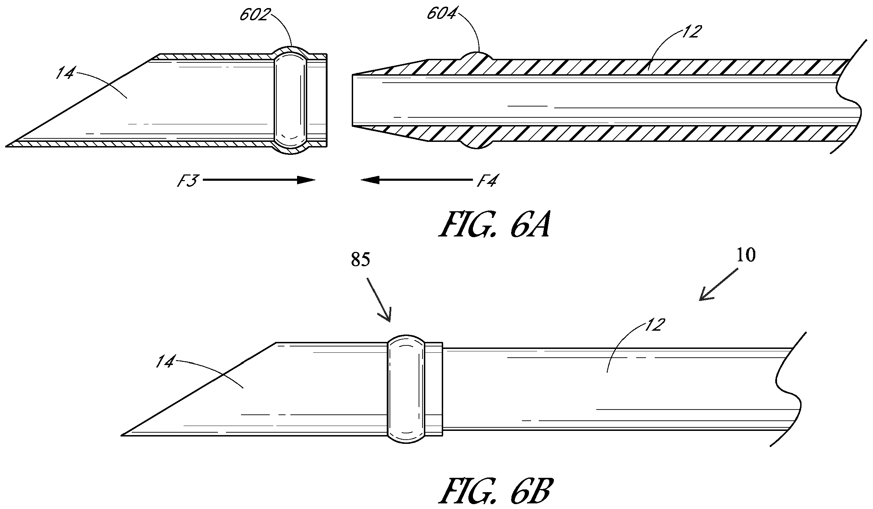

In yet another embodiment as particularly shown in Figures 6a, the needle shaft 12 may be provided with a projection or bump 604. The bump 604 can be located adjacent to the distal end of the needle shaft 12. The bump 604 can embody a raised ring or two or more separate bumps. In an example, the needle tip 14 includes an internal complementary recess 602. The recess 602 extends in a circumferential ring around an internal surface of the tip 14 and is preferably located at the proximal end of needle tip 14. Preferably, the recess 602 is formed to fit over the bump 604 of the needle shaft 12. To couple the needle shaft 12 with needle tip 14 to form needle assembly 10 as particularly shown in Figure 6b, the two components are forced toward each other, in direction F3 and F4 respectively as shown in Figure 6a, until the bump 604 is located within the recess 602, as shown in Figure 6b thereby forming a coupling 85 to attach the needle tip 14 with the needle shaft 12. The bump 604 is retained within the recess 602 by friction or interference fit during use of the needle 10. Once joined, the recess 602 can function as a change in profile similar to a crimp for engaging a needle guard, as previously discussed.

The bump 604 and the shaft 12 can be made from a relatively less rigid material, such as a plastic material, than the material used to form the needle tip 14, such as a metal material or a plastic material coated or plated with harder material, so that when the opposing forces are applied, the shaft 12 can deform and fit into the opening of the needle tip 14. As shown, the distal end of the shaft 12 can be tapered to facilitate insertion into the needle tip.

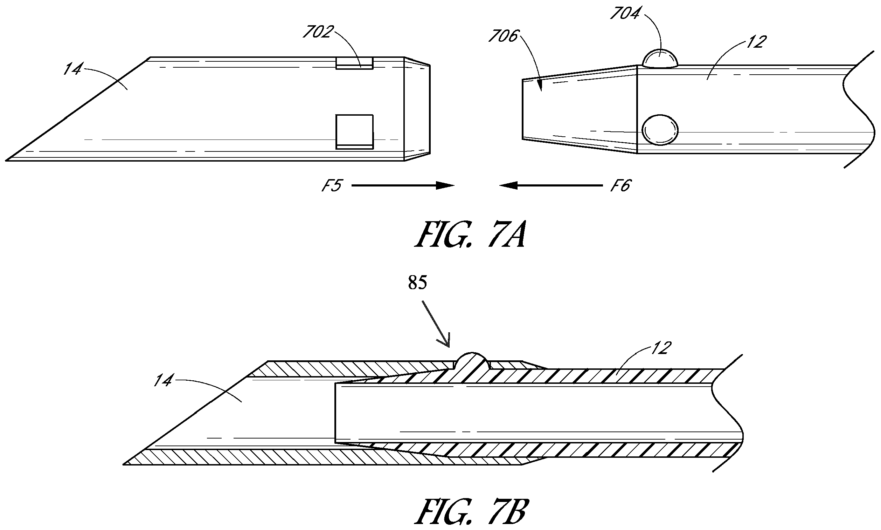

Of course, the design of the bump and the corresponding recess or indentation may be of different shapes, such as a circumferential rib or one or more protrusions. For example, as particularly shown in Figure 7a, the needle shaft 12 includes a plurality of protrusions or bumps 704. The bumps 704 are preferably located at or near the distal end 706 of the needle shaft 12. In the present embodiment, the needle tip 14 is provided with a complementary aperture 702 for each bump 704, preferably located at the proximal end of needle tip 14. Preferably, the aperture 702 is formed to receive the bump 704 of the needle shaft 12. To couple the needle shaft 12 with needle tip 14 to form needle assembly 10 as particularly shown in Figure 7b, the two components are forced toward each other, in directions F5 and F6 respectively as shown in Figure 7a, until the bumps 704 are located within the apertures 702 as shown in Figure 7b thereby forming a coupling 85 to attach the needle tip 14 with the needle shaft 12. The bumps 704 may be arranged with irregular spacing so that the needle tip 14 and shaft 12 can only be fitted together in a single orientation. As shown, the distal end of the shaft 12 can be tapered to facilitate insertion into the needle tip.

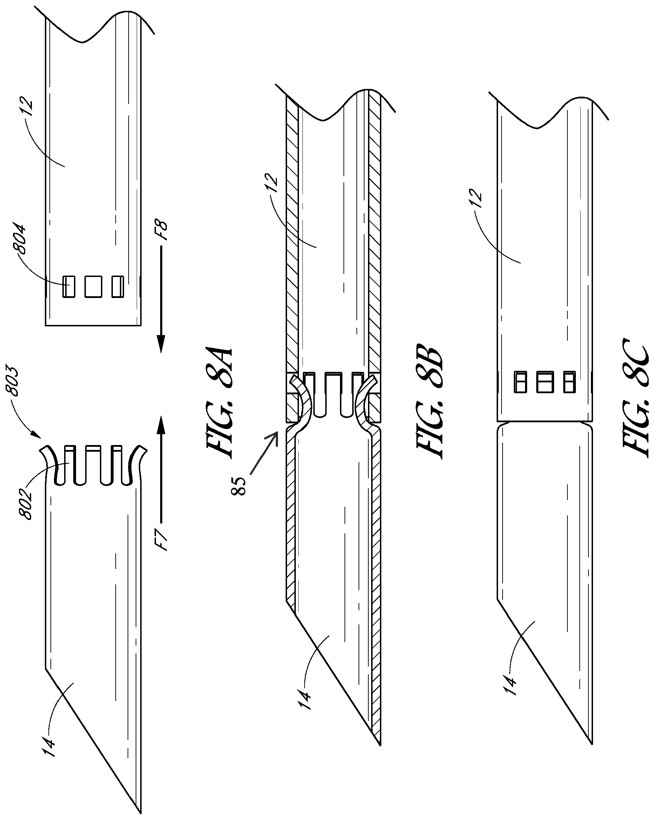

In yet another embodiment as particularly shown in Figure 8a, the needle shaft 12 includes one or more holes 804. The one or more holes 804 are preferably located at or near the distal end of the needle shaft 12. In this embodiment, the needle tip 14 includes one or more flared fingers 802. The flared fingers 802 extend proximally from the proximal end 803 of needle tip 14. Preferably, the flared fingers 802 are formed to fit within the one or more respective holes 804 of the needle shaft 12. To couple the needle shaft 12 with needle tip 14 to form needle assembly 10 as particularly shown in Figure 8c, the two components are forced toward each other, in directions F7 and F8 respectively as shown in Figure 8a, until the one or more flared fingers 802 are located within respective ones of the holes 802 as shown in Figure 8b thereby forming a coupling 85 to attach the needle tip 14 with the needle shaft 12. Side-to-side motions or wiggling motions may be employed to get the flared fingers 802 to fit inside the distal opening of the shaft 12.

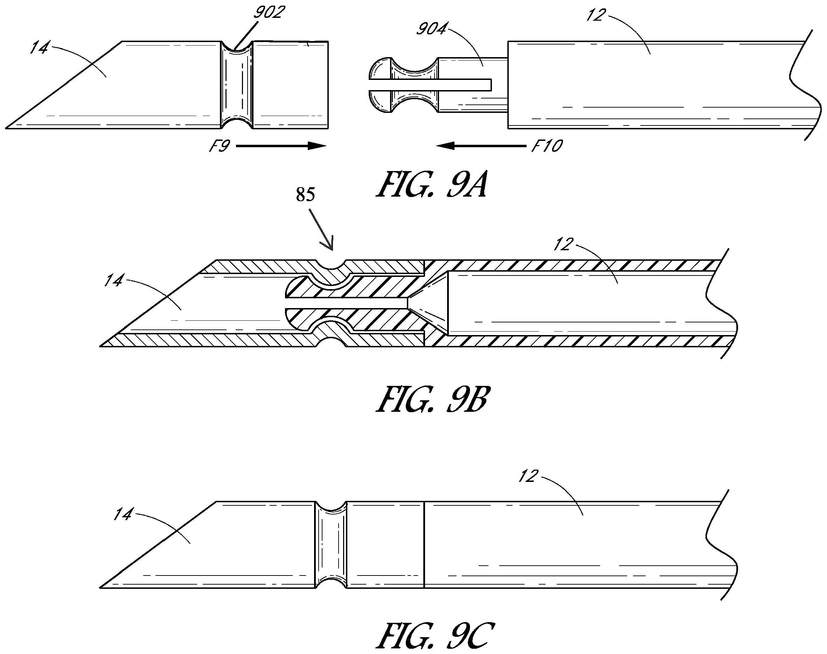

In yet another embodiment as particularly shown in Figure 9a, the needle shaft 12 includes an arm 904, which can include a pair of tines or prongs having a gap therebetween and each including a barbed end. The arm 904 is preferably located at the distal end of the needle shaft 12. In this embodiment, the needle tip 14 includes a metal rib 902 extending inwardly of the needle tip 14, such as an annular recess. The metal rib 902 is located near the proximal end of needle tip 14. Preferably, the arm 904 is formed to flex into the rib 902. This is achieved by forming the arm 904 from two opposed halves biased into the position shown in Figure 9a. As the arm 904 is pushed through the rib 902, the arm moving in direction F10 and the rib in direction F9, the opposed halves of the arm 904 are pushed together, creating an outward bias that swing radially once the depression in the halves of the arm 904 are aligned with the rib 902 thereby coupling the shaft 12 to the needle tip 12. The connection defines a coupling 85 to attach the needle tip 14 with the needle shaft 12.

Figures 13A to 13D show alternative attachment mechanisms. In Figure 13A, the needle shaft 1306 extends the full length of the needle 1302, from the proximal end to the needle tip. A first material, which can be a metal material, can then be deposited over the needle shaft 1306 to form the needle tip 1304. The portion 1300 of the shaft 1306 proximal of the bevel may be formed from the same material as the needle shaft 1306, or may be formed from a heat resistant or heat tolerant material such as a silicon composition, that is relatively insusceptible to deformation during deposition of the first material during formation of the needle tip 1304. The second material may alternatively be over-moulded, or under-moulded as the case may be, into the needle tip 1304.

As shown in Figure 13B, a needle having a predetermined length and two needle tips 1308, 1310 may be formed concurrently from a body of a first material 1312 by depositing the first material onto a length of tubing 1314. The second material may alternatively be over-moulded into the first body of material 1312. The two needle tips 1308, 1310 are then formed by cutting through the first material 1312 diagonally, e.g., along line D-D as shown in Figure 13B.

In an alternative embodiment shown in Figure 13C, the shaft 1316 is formed around a tube 1318, or the tube 1318 is inserted into the shaft 1316. A first material is then deposited over the shaft 1316 to form the needle tip 1320. This enables the first material to mask the front of the shaft at region 1322. The tip can then be machined to ensure that a sharp bevel 1324 is provided. Again, the second material may alternatively be moulded or deposited into the needle tip 1320, with the needle tip 1320 being pre-formed to have the shape as shown.

Figures 13A to 13C illustrate embodiments where the first material is deposited over the second material to form a needle tip on a pre-existing shaft. The second material can be a substrate upon which the first material is deposited or moulded over. In some cases, an intermediate material is formed over the second material to act as an interface between the first and second materials during the depositing step of the first material. Thus, the embodiment with the intermediate material may be considered a needle embodiment with three material layers, the inner material or second material, the intermediate layer, and the outer-most material or first material. This interface may bond to the second material and have higher heat tolerance when compared with the second material to facilitate deposition of the first material onto the intermediate layer. Alternatively, the intermediate material may be bonded to the second material and be electrically conductive to facilitate electrolytic deposition of the first material over the intermediate material.

The integral needle tip form, whether formed from a substrate independently of the needle shaft or whether it be part of the needle shaft, may be heated so that the first material is at least partially embedded in the second material during depositing of the first material over the substrate. The first material can bind to the second material during cooling or curing of the needle tip after depositing the first material. Preferably, depositing the first material over the needle tip should include leaving a portion of the needle tip exposed to form a window for viewing primary flashback. For example, the inner first material forming the substrate can be transparent or semi-opaque and an opening or aperture may be located anywhere on the needle tip when depositing the first material. In an example, the window can be located near the proximal end of the needle tip where the needle tip connects to, or transitions into, the needle shaft. This may be achieved by applying a mask over a portion of the needle tip form so that the first material is not deposited over the masked area. In this embodiment, exposed is understood to mean "not covered with the first material" or "masked" or "formed by masking a portion of the second material during deposition".

The above-mentioned needle has a needle shaft which comprises an integral needle tip form at a distal end of the needle shaft serving as a substrate. The needle tip 14 of the final needle is attached to the needle shaft 12 by depositing the first material over the integral needle tip form of the substrate. To reduce the likelihood that the first material will unsheathe from the substrate or needle shaft, the needle shaft may comprise a depression or protrusion, as shown in Figures 2 and 6a, and the first material may be deposited over a length of the needle shaft from, and including, the bevel to at least the depression or protrusion. Similarly, the second material may be over-moulded into a needle tip comprising a depression or protrusion to fill that depressed or surround the protrusion and be cured to form a shaft secured to the tip.

Advantageously, the one material being formed over a depression or protrusion in the other means that the two will grip in the event of force that may otherwise unsheathe the first material from the second material.

An alternative formation method is shown in Figure 13D. In the embodiment shown, a pre-formed needle tip 1326 is provided and a tube 1330 is inserted into the pre-formed tip 1326. A cavity 1328, which can be generally cylindrical in shape, is thus created between the tube 1330 and the pre-formed tip 1326. The second material is then flowed, injected or otherwise driven or deposited into the cavity 1328 to form the needle shaft, such as by using the over-moulding technique described elsewhere herein.

As shown in FIG. 13D, the pre-formed needle tip 1326 may include one or more projections 1332. The projections 1332 can be directed inwardly into or towards the cavity 1328. The projections 1332 may extend radially inwardly. The projections 1332 may each be formed as a circumferential rib or as individual projections spaced around the circumference of the pre-formed needle tip 1326. The projections can embody spirally formed or helix rib material formed inwardly from the outer surface of the pre-formed needle tip. The projections 1332 prevent the pre-formed tip 1326 from sliding off the distal end of the completed needle shaft after formation of the needle shaft.

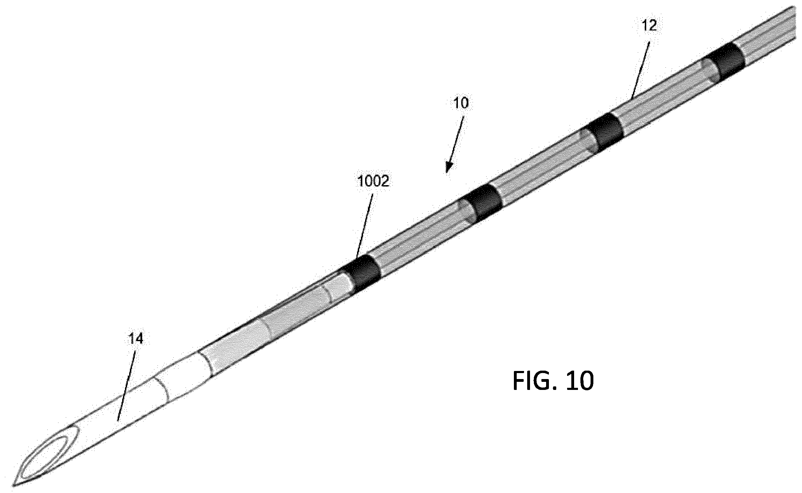

In a further embodiment, the needle assembly 10 includes echogenic patterns or strips 1002 as particularly shown in Figure 10. The strips 1002 reflect ultrasound signals to provide an indication of the location of the needle 10 during ultrasound guided insertion. The echogenicity patterns or strips may be provided on the shaft or on the needle tip 14 for ultrasound guided insertion of a peripheral intravenous catheter (PIVC).

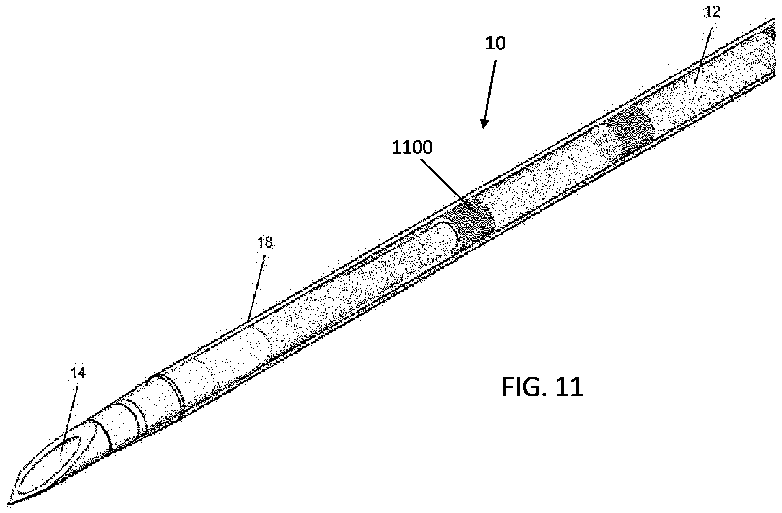

Figure 11 shows the needle assembly 10 of Figure 10, with a catheter 18 located over the needle 10. The catheter 18 can attach to a catheter hub via a ferrule or bushing. The catheter hub can optionally include a valve, a valve opener, and a needle guard in the hollow interior of the catheter hub. In this embodiment, echogenic strips 1100 may also serve as the indicia when exposed to blood, similar to indicia 32 of Figure 12, whereby the strips 1100 are visible during primary flashback and are obscured or obstructed by the blood in the catheter during secondary flashback.

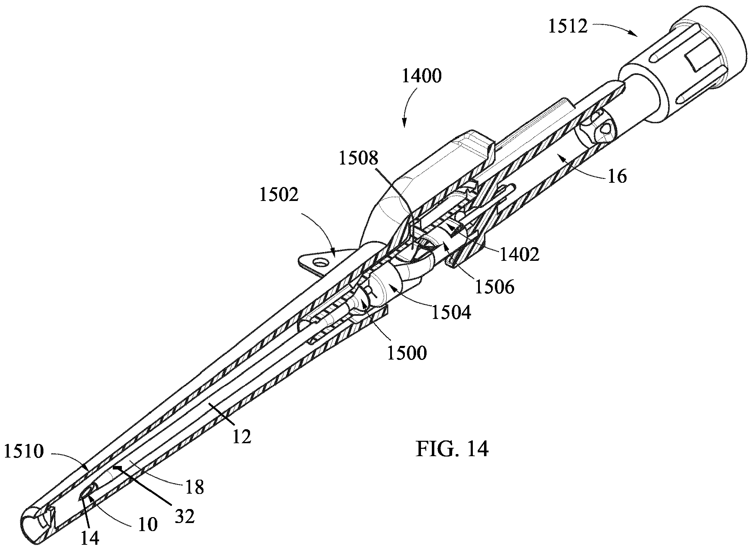

Figure 14 is a schematic cross-sectional perspective view showing a catheter assembly 1400 incorporating a needle 10 as described with reference to Figures 1 to 13. The catheter assembly 14 comprises a needle 10, a catheter hub 1402 and a catheter 18. The catheter 18 is connected to the catheter hub 1402, such as with a ferrule 1500, and surrounds the needle 10. The catheter hub 1402 is shown with a wing 1502. The catheter assembly 1400 can be used in the standard manner. A valve 1504, a valve opener 1506 for opening one or more flaps on the valve 1504, such as three flaps, and a needle guard 1508 may be located in the interior space of the catheter hub 1402, as described in U.S. Pub. No. 2018/0214682. Also shown is a removable protective cap 1510 and a gas or air vent 1512.

The catheter 18 can be transparent or translucent. A marking or indicia 32 can be located on the needle 10 to be obscured by bodily fluid during secondary flashback, as previously discussed.

In some embodiments, the catheter assembly 1400 includes a conduit or port for coupling a valve for selectably fluidly connecting the catheter to a receptacle. For example, the catheter hub can be an integrated catheter having a port, a tubing attached to the port, and a needleless connector connected to the opposite end of the tubing. The catheter hub can alternatively be a ported catheter in which a port can receive a needle tip or a male Luer tip directly without the tubing. The catheter assembly may also include a needle guard for guarding the needle tip 14 after use, to prevent needle stick injury, as described in U.S. Pub. No. 2018/0214682.

In another embodiment, the catheter assembly 1400 further includes a blood septum control mechanism. Preferably, the blood septum control mechanism is a valve having a plurality of slits defining a plurality of flaps. The valve can be selectably in an open or closed position. For example, the valve flaps of the valve close the valve when the needle is retracted to stop blood from flowing out of the catheter hub and the flaps open the valve when a coupling with an external receptacle is made, such as when a needle tip or an IV adaptor is inserted into the proximal opening of the catheter hub, which can embody a female Luer or a threaded female Luer.

Of course, the shape of the needle shaft 12 can be hollow, solid or semi circle, for example. The profile of the needle shaft 12 can be suited to match the requirements of the particular application. Advantageously, for embodiments where the needle shaft 12 is solid, the manufacturing of the needle shaft 12 may be easier and cheaper than that of a hollow needle shaft, such as by using moulding to form the shaft. However, the drawback of such a configuration is that secondary flashback may not be visualized. Primary flashback may be visualized by including a notch or groove on the needle shaft 12, such as aa longitudinal (i.e., extending along the needle) groove extending from a location at or near the needle tip to a location outside the patient during insertion of the needle.

Advantageously, embodiments of the invention enable low puncture force penetration of a patient while providing high flashback visibility via visual feedback through the surface layer of the needle. Embodiments of the invention incorporate plastic within the needle thereby lowering the proportion of metal in the needle and reducing cost. Using both plastic and metal also provides the manufacturer with flexibility on how to form the needle. Maintaining the needle tip as metal advantageously results in less discomfort in subjects.

In addition, in some embodiments, some portions of the needle, being plastic, can be transparent. Using transparent plastic facilitates visibility of primary flashback in the needle shaft without requiring either a window/notch to be formed in the needle, or blood to exit the proximal end of the needle.

Advantageously, embodiments of the invention provide solutions to the space limitations in a catheter, such as an intravenous catheter. Some features which typically heavily rely on the plastic catheter hub of conventional systems result in severe space limitations. Embodiments of this invention allow such features to be transferred to the plastic needle 12 instead. Said differently, the needle shaft can be moulded with a reduced diameter section to then provide space for other components to make use of the space vacated by the reduced diameter needle section. Examples of such features include the blood control septum, safety mechanism such as the safety clip minimum diameter.

Throughout this specification, unless the context requires otherwise, the word "comprise", and variations such as "comprises" and "comprising", will be understood to imply the inclusion of a stated integer or step or group of integers or steps but not the exclusion of any other integer or step or group of integers or steps. The words having or including can have similar meaning. Moreover, features from different embodiments may be combined with other embodiments, features may be substituted for one another or removed, without departing from the present teachings.

The reference to any prior art in this specification is not, and should not be taken as, an acknowledgment or any form of suggestion that the prior art forms part of the common general knowledge.

Use of "first" and "second" are intended to distinguish between two different items or components only and not intended to be limiting, other than to distinguish between two different items or components, unless the context indicates otherwise.

Methods of making and of using multi-piece needles and the catheter assemblies and components thereof are not claimed.

Although limited embodiments of needles and catheter assemblies and their components have been specifically described and illustrated herein, many modifications and variations will be apparent to those skilled in the art. Accordingly, it is to be understood that the needles, catheter assemblies, and their components constructed according to principles of the disclosed devices, systems, and methods may be embodied other than as specifically described herein. The invention is determined by the appended claims.

## Claims

1. A catheter assembly (1400) comprising:
a needle (10);
a catheter hub (1402); and
a catheter (18) connected to the catheter hub (1402) and surrounding the needle (10), wherein the needle (10) comprises:
a needle tip (14) formed from a first rigid material, the needle tip (14) comprising a bevel (30) for puncturing a subject; and
a needle shaft (12) comprising a second material,
wherein the needle tip (14) is separately formed and subsequently attached to the needle shaft (12) to form the needle (10);
**characterized in that**
the second material is a light permeable material to enable visible detection of primary flashback in the needle shaft (12).

2. The catheter assembly (1400) of claim 1, wherein the first material has higher rigidity than the second material.

3. The catheter assembly (1400) of claim 1 or 2, wherein the first material is a metal and/or the second material is a plastic.

4. The catheter assembly (1400) of any one of the preceding claims, wherein the second material is transparent or translucent.

5. The catheter assembly (1400) of any one of the preceding claims, wherein the needle tip (14) is attached to the needle shaft (12) through a coupling (85), wherein the coupling (85) preferably comprises:
one or more indentations on one of the needle shaft (12) and the needle tip (14); and, for each indentation,
a protrusion on the other one of the needle shaft (12) and the needle tip (14), and arranged to be received in the indentation, thereby to couple the needle tip (14) to the needle shaft (12) and/or the coupling (85) preferably comprises:
one or more apertures (702) on one of the needle shaft (12) and the needle tip (14); and
for each aperture (702), a protrusion (704) on the other one of the needle shaft (12) and the needle tip (14), and arranged to be received in the aperture (702), thereby to couple the needle tip (14) to the needle shaft (12).

6. The catheter assembly (1400) of any one of the preceding claims, wherein the needle tip (14) comprises:
the first material having the bevel (30); and
either:
(i) the second material is formed onto the first material, thereby to form the needle shaft (12); or
(ii) the needle shaft (12) is formed from the second material and thereafter connected to the first material.

7. The catheter assembly (1400) of any one of the preceding claims, wherein the needle shaft (12) comprises an integral needle tip (14) at a distal end of the needle shaft (12), and the needle tip (14) is attached to the needle shaft (14) by depositing the first material over an integral needle tip form (1306).

8. The catheter assembly (1400) of any one of the preceding claims, wherein the needle tip (14) comprises:
a proximal end comprising one of a depression and protrusion; and
wherein forming the second material onto the needle tip (14) comprises depositing the second material over a length of the needle tip (14) from a position distal of the depression or protrusion to, and past, the proximal end of the needle tip (14).

9. The catheter assembly (1400) of any one of claims 7 or 8, further comprising an intermediate material formed on, or attached to, the integral needle tip form for heat dissipation during depositing the first material.

10. The catheter assembly (1400) of any one of claims 6 to 9, wherein the second material is formed by moulding onto the first material and/or the first material is bound to the second material during cooling or curing of the second material.

11. The catheter assembly (1400) of any one of claims 6 to 10, wherein the needle tip comprises an aperture to form a window for viewing primary flashback.

12. The catheter assembly (1400) of any one of the preceding claims further comprising:
a needle hub (16); and
a tether (506) connected between the needle tip (14) and needle hub (16), to couple the needle tip (14) to the needle hub (16).

13. The catheter assembly (1400) of any one of the preceding claims, wherein the needle shaft (12) comprises a marking (32), the marking (32) being located on the needle shaft (12) to be visible during primary flashback.

14. The catheter assembly (1400) according to claim 13, wherein the catheter (18) is transparent or translucent and the marking (32) is obscured by bodily fluid between the catheter and needle shaft during secondary flashback.

15. The catheter assembly (1400) according to any one of the preceding claims, wherein the needle hub (16) is integrally formed with the needle (10).

## Patentansprüche

1. Katheteranordnung (1400), aufweisend:
eine Nadel (10);
einen Katheteransatz (1402); und
einen Katheter (18), der mit dem Katheteransatz (1402) verbunden ist und die Nadel (10) umgibt, wobei die Nadel (10) aufweist:
eine Nadelspitze (14), die aus einem ersten starren Material gebildet ist, die Nadelspitze (14) aufweisend eine Abschrägung (30) zum Punktieren eines Subjekts; und
einen Nadelschaft (12), aufweisend ein zweites Material,
wobei die Nadelspitze (14) separat gebildet und anschließend an dem Nadelschaft (12) befestigt wird, um die Nadel (10) zu bilden;
**dadurch gekennzeichnet, dass**
das zweite Material ein lichtdurchlässiges Material ist, um eine visuelle Erkennung eines primären Rückflusses in dem Nadelschaft (12) zu ermöglichen.

2. Katheteranordnung (1400) nach Anspruch 1, wobei das erste Material eine höhere Steifigkeit als das zweite Material aufweist.

3. Katheteranordnung (1400) nach Anspruch 1 oder 2, wobei das erste Material ein Metall ist und/oder das zweite Material ein Kunststoff ist.

4. Katheteranordnung (1400) nach einem der vorstehenden Ansprüche, wobei das zweite Material transparent oder durchscheinend ist.

5. Katheteranordnung (1400) nach einem der vorstehenden Ansprüche, wobei die Nadelspitze (14) über ein Koppeln (85) an dem Nadelschaft (12) befestigt ist, wobei das Koppeln (85) vorzugsweise aufweist:
eine oder mehrere Vertiefungen an eines von dem Nadelschaft (12) und der Nadelspitze (14); und, für jede Vertiefung,
einen Vorsprung an dem anderen einen des Nadelschafts (12) und der Nadelspitze (14) und der eingerichtet ist, um in der Vertiefung aufgenommen zu werden, um dadurch die Nadelspitze (14) mit dem Nadelschaft (12) zu koppeln, und/oder das Koppeln (85) vorzugsweise aufweist:
eine oder mehrere Öffnungen (702) an eines von dem Nadelschaft (12) und der Nadelspitze (14); und
für jede Öffnung (702) einen Vorsprung (704) an dem anderen einen des Nadelschafts (12) und der Nadelspitze (14) und der eingerichtet ist, um in der Öffnung (702) aufgenommen zu werden, um dadurch die Nadelspitze (14) mit dem Nadelschaft (12) zu koppeln.

6. Katheteranordnung (1400) nach einem der vorstehenden Ansprüche, wobei die Nadelspitze (14) aufweist:
das erste Material, das die Abschrägung (30) aufweist; und
entweder:
(i) das zweite Material auf das erste Material aufgebracht wird, um dadurch den Nadelschaft (12) zu bilden; oder
(ii) der Nadelschaft (12) aus dem zweiten Material gebildet wird und danach mit dem ersten Material verbunden wird.

7. Katheteranordnung (1400) nach einem der vorstehenden Ansprüche, wobei der Nadelschaft (12) eine integrierte Nadelspitze (14) an einem distalen Ende des Nadelschafts (12) aufweist und die Nadelspitze (14) durch Ablagern des ersten Materials über einer integrierten Nadelspitzenform (1306) an dem Nadelschaft (14) befestigt ist.

8. Katheteranordnung (1400) nach einem der vorstehenden Ansprüche, wobei die Nadelspitze (14) aufweist:
ein proximales Ende, aufweisend eines von einer Vertiefung und einem Vorsprung; und
wobei das Bilden des zweiten Materials auf die Nadelspitze (14) das Ablagern des zweiten Materials über eine Länge der Nadelspitze (14) von einer Position distal der Vertiefung oder des Vorsprungs zu dem proximalen Ende der Nadelspitze (14) und darüber hinaus aufweist.

9. Katheteranordnung (1400) nach einem der Ansprüche 7 oder 8, ferner aufweisend ein Zwischenmaterial, das auf der integrierten Nadelspitzenform gebildet oder an dieser befestigt ist, für eine Wärmeableitung während des Ablagerns des ersten Materials.

10. Katheteranordnung (1400) nach einem der Ansprüche 6 bis 9, wobei das zweite Material durch Formen auf dem ersten Material gebildet wird und/oder das erste Material während eines Abkühlens oder Aushärtens des zweiten Materials mit dem zweiten Material gebunden wird.

11. Katheteranordnung (1400) nach einem der Ansprüche 6 bis 10, wobei die Nadelspitze eine Öffnung aufweist, um ein Fenster zum Betrachten von primärem Rückfluss zu bilden.

12. Katheteranordnung (1400) nach einem der vorstehenden Ansprüche, ferner aufweisend :
einen Nadelansatz (16); und
ein Halteband (506), das zwischen der Nadelspitze (14) und dem Nadelansatz (16) verbunden ist, um die Nadelspitze (14) mit dem Nadelansatz (16) zu koppeln.

13. Katheteranordnung (1400) nach einem der vorstehenden Ansprüche, wobei der Nadelschaft (12) eine Markierung (32) aufweist, wobei die Markierung (32) auf dem Nadelschaft (12) gelegen ist, um während des primären Rückflusses sichtbar zu sein.

14. Katheteranordnung (1400) nach Anspruch 13, wobei der Katheter (18) transparent oder durchscheinend ist und die Markierung (32) während eines sekundären Rückflusses durch Körperflüssigkeit zwischen dem Katheter und dem Nadelschaft verdeckt wird.

15. Katheteranordnung (1400) nach einem der vorstehenden Ansprüche, wobei der Nadelansatz (16) mit der Nadel (10) einstückig gebildet ist.

## Revendications

1. Ensemble cathéter (1400) comprenant :
une aiguille (10) ;
un moyeu de cathéter (1402) ; et
un cathéter (18) raccordé au moyeu de cathéter (1402) et entourant l'aiguille (10), dans lequel l'aiguille (10) comprend :
une pointe d'aiguille (14) formée à partir d'un premier matériau rigide, la pointe d'aiguille (14) comprenant un biseau (30) pour perforer un sujet ; et
une tige d'aiguille (12) comprenant un second matériau,
dans lequel la pointe de l'aiguille (14) est formée séparément puis fixée à la tige de l'aiguille (12) pour former l'aiguille (10) ;
**caractérisé en ce que**
le second matériau est un matériau perméable à la lumière pour permettre la détection visible du retour de flamme primaire dans le corps de l'aiguille (12).

2. Ensemble cathéter (1400) selon la revendication 1, dans lequel le premier matériau a une rigidité supérieure au second matériau.

3. Ensemble cathéter (1400) selon la revendication 1 ou 2, dans lequel le premier matériau est un métal et/ou le second matériau est un plastique.

4. Ensemble cathéter (1400) selon l'une quelconque des revendications précédentes, dans lequel le second matériau est transparent ou translucide.

5. Ensemble cathéter (1400) selon l'une quelconque des revendications précédentes, dans lequel la pointe de l'aiguille (14) est fixée à la tige de l'aiguille (12) par l'intermédiaire d'un accouplement (85), dans lequel l'accouplement (85) comprend de préférence :
une ou plusieurs indentations sur la tige de l'aiguille (12) et sur la pointe de l'aiguille (14) ; et, pour chaque indentation,
une protubérance sur l'autre tige d'aiguille (12) et la pointe d'aiguille (14), et agencée pour être reçue dans l'indentation, afin d'accoupler la pointe d'aiguille (14) à la tige d'aiguille (12) et/ou l'accouplement (85) comprend de préférence :
une ou plusieurs ouvertures (702) sur la tige de l'aiguille (12) et sur la pointe de l'aiguille (14) ; et pour chaque ouverture (702), une protubérance (704) sur l'autre partie de la tige de l'aiguille (12) et la pointe de l'aiguille (14), et agencée pour être reçue dans l'ouverture (702), afin d'accoupler la pointe de l'aiguille (14) à la tige de l'aiguille (12).

6. Ensemble cathéter (1400) selon l'une quelconque des revendications précédentes, dans lequel la pointe de l'aiguille (14) comprend :
le premier matériau est doté d'un biseau (30) ; et
soit :
(i) le second matériau est formé sur le premier matériau, formant ainsi la tige de l'aiguille (12) ; ou
(ii) la tige de l'aiguille (12) est formée à partir du second matériau et est ensuite raccordée au premier matériau.

7. Ensemble cathéter (1400) selon l'une quelconque des revendications précédentes, dans lequel la tige d'aiguille (12) comprend une pointe d'aiguille intégrée (14) à une extrémité distale de la tige d'aiguille (12), et la pointe d'aiguille (14) est fixée à la tige d'aiguille (14) en déposant le premier matériau sur une forme de pointe d'aiguille intégrée (1306).

8. Ensemble cathéter (1400) selon l'une quelconque des revendications précédentes, dans lequel la pointe de l'aiguille (14) comprend :
une extrémité proximale comprenant une dépression et une protubérance ; et
dans lequel la formation du second matériau sur la pointe de l'aiguille (14) comprend le dépôt du second matériau sur une longueur de la pointe de l'aiguille (14) à partir d'une position distale de la dépression ou de la protubérance jusqu'à l'extrémité proximale de la pointe de l'aiguille (14) et au-delà de celle-ci.

9. Ensemble cathéter (1400) selon l'une quelconque des revendications 7 ou 8, comprenant en outre un matériau intermédiaire formé sur, ou attaché à, la forme intégrale de la pointe de l'aiguille pour la dissipation de la chaleur pendant le dépôt du premier matériau.

10. Ensemble cathéter (1400) selon l'une quelconque des revendications 6 à 9, dans lequel le second matériau est formé par moulage sur le premier matériau et/ou le premier matériau est lié au second matériau pendant le refroidissement ou le durcissement du second matériau.

11. Ensemble cathéter (1400) selon l'une quelconque des revendications 6 à 10, dans lequel la pointe de l'aiguille comprend une ouverture pour former une fenêtre permettant d'observer le retour de flamme primaire.

12. Ensemble cathéter (1400) selon l'une quelconque des revendications précédentes, comprenant en outre : un moyeu d'aiguille (16) ; et
un filin (506) raccordé entre la pointe de l'aiguille (14) et le moyeu d'aiguille (16), pour accoupler la pointe de l'aiguille (14) au moyeu d'aiguille (16).

13. Ensemble cathéter (1400) selon l'une quelconque des revendications précédentes, dans lequel la tige d'aiguille (12) comprend un marquage (32), le marquage (32) étant situé sur la tige d'aiguille (12) pour être visible pendant le retour de flamme primaire.

14. Ensemble cathéter (1400) selon la revendication 13, dans lequel le cathéter (18) est transparent ou translucide et le marquage (32) est masqué par le fluide corporel entre le cathéter et la tige de l'aiguille pendant le retour de flamme secondaire.

15. Ensemble cathéter (1400) selon l'une quelconque des revendications précédentes, dans lequel le moyeu d'aiguille (16) est formé d'une seule pièce avec l'aiguille (10).
